# EUROPEAN PATENT APPLICATION

(11) **EP 3 542 822 A1**
(43) Date of publication of application: **25.09.2019**
(21) Application number: 17871400.2
(22) Date of filing: 16.11.2017
(51) Int. Cl.: A61K 45/00, A61K 9/127, A61K 31/05, A61K 35/34, A61K 47/24, A61K 47/42, A61P 9/04, C12N 5/074

(54) **METHOD FOR PRODUCING MYOCARDIAL STEM CELL USED FOR TREATMENT AND/OR PREVENTION OF CARDIAC ARREST**

(30) Priority: 16.11.2016 JP 2016223069
(71) Applicant: Luca Science Inc., Chuo-ku Tokyo 103-0023 (JP)
(72) Inventor: HARASHIMA Hideyoshi, Sapporo-shi Hokkaido 060-0808 (JP); YAMADA Yuma, Sapporo-shi Hokkaido 060-0808 (JP); ABE Jiro, Sapporo-shi Hokkaido 060-0808 (JP); TAKEDA Atsuhito, Sapporo-shi Hokkaido 060-0808 (JP)
(74) Representative: J A Kemp
(86) International application number: PCT/JP2017/041250
(87) International publication number: WO 2018/092839

(57) **Abstract**

[Problem to be Solved]

An object of the present invention is to provide a novel myocardial stem cell for transplantation which enables maintenance of a therapeutic effect on and/or preventive effect against cardiac failure for a prolonged period, a method for producing the myocardial stem cell, and a cell preparation containing the myocardial stem cell.

[Solution]

The present invention relates to a method for producing a myocardial stem cell to be used for treatment and/or prevention of cardiac failure, the method comprising the step of introducing a complex of a mitochondria-targeting carrier and a mitochondria activating agent into a myocardial stem cell; a cell produced by the method and a cell preparation containing the cell; and a liposome to be used for producing the cell. According to the present invention, there can be provided a myocardial stem cell which is capable of maintaining a therapeutic effect and/or preventive effect by cell transplantation for a prolonged period.

SEQUENCE LISTING

<110>  National Corporation University Hokkaido University

<120>  Method for producing cardiac progenitor cells for use in treatment
and/or prevention of cardiac failure

<130>  P16HU04WO

<160>  2

<170>  PatentIn version 3.5

<210>  1
<211>  8
<212>  PRT
<213>  Artificial Sequence

<220>
<223>  Designed mitochondrial targeting peptide

<220>
<221>  misc_feature
<222>  (1)
<223>  Xaa stands for D-Arg

<220>
<221>  misc_feature
<222>  (2)
<223>  Xaa stands for dimethyl tyrosine

<220>
<221>  misc_feature
<222>  (5)
<223>  Xaa stands for D-Arg

<220>
<221>  misc_feature
<222>  (6)
<223>  Xaa stands for dimethyl tyrosine

<400>  1

Xaa Xaa Lys Phe Xaa Xaa Lys Phe
1               5


<210>  2
<211>  8
<212>  PRT
<213>  Artificial Sequence

<220>
<223>  Octa-arginine

<400>  1

Arg Arg Arg Arg Arg Arg Arg Arg
1               5

## Description

### Technical Field

The present invention relates to a method for producing a novel myocardial stem cell for use in treatment and/or prevention of cardiac failure, a cell population including myocardial stem cells having activated mitochondria, a cell preparation for use in treatment and/or prevention of cardiac failure containing the myocardial stem cell or the cell population, and a liposome for use in producing the myocardial stem cell.

### Background Art

The cardiac failure refers to a symptom in which a heart-related disease such as myocardial infarction, cardiomyopathy or angina pectoris, or a disease other than a heart-related disease, such as hypertension, kidney disease or a side effect of chemotherapy against a malignant tumor causes deterioration of the cardiac function, so that a necessary amount of blood cannot be supplied to the lung or throughout the body. The cardiac failure is a second main cause of death behind cancer in Japan, and fundamental methods for treatment of cardiac failure include heart transplantation. However, the heart transplantation has various disadvantages such as a chronic shortage of transplant donors, limits of service life of transplanted organs, rejection, oral administration of immunosuppressants throughout life, and frequent hospitalization for catheter tests.

Studies have been conducted on cell transplantation therapy using various cells including iPS cells since cell transplantation was proposed as a promising method for treatment of cardiac failure in the late 2000s. In particular, myocardial stem cell transplantation has the following advantages: it is immunologically safe because it is transplantation using self-somatic cells; and it can be carried out by a low-invasive method. The myocardial stem cell transplantation has been shown to have a certain effect in clinical trials (e.g. Non Patent Literatures 1, 2 and 3).

The myocardial stem cell transplantation has, for example, the following disadvantages: the transplanted cell engraftment effect is limited in myocardial stem cell transplantation experiments with a pig ischemic reperfusion model (Non Patent Literature 4); and the viability improvement effect is limited in myocardial stem cell transplantation experiments with a rat doxorubicin cardiomyopathy model (Non Patent Literature 5). Thus, maintenance of the therapeutic effect for a prolonged period is one of forthcoming challenges in myocardial stem cell transplantation.

### Citation List

### Non Patent Literature

Non Patent Literature 1: Bolli, R. et al., Lancet 2011, 378, pp. 1847-1857
Non Patent Literature 2: Makkar, R. R. et al., Lancet 2012, 379, pp. 895-904
Non Patent Literature 3: Ishigami, S. et al., Circulation research 2015, 116, pp. 653-664
Non Patent Literature 4: Takehara, N. et al., J. Am. Coll. Cardiol. 2008, 52, pp. 1858-65
Non Patent Literature 5: De Angelis, A. et al., Circulation 2010, 121, pp. 276-292

### Disclosure of Invention

### Technical Problem

An object of the present invention is to provide a novel myocardial stem cell for use in transplantation which enables maintenance of a therapeutic effect on and/or preventive effect against cardiac failure for a prolonged period, a method for producing the myocardial stem cell, and a cell preparation containing the myocardial stem cell.

### Solution to Problem

The present inventors have found that by delivering a mitochondria activating agent to mitochondria of a myocardial stem cell, a cell for use in transplantation can be produced which has an enhanced engraftment effect, and maintains a therapeutic effect for a prolonged period, leading to completion of the following inventions.

(1) A method for producing a myocardial stem cell for use in treatment and/or prevention of cardiac failure, the method comprising the step of introducing a complex of a mitochondria-targeting carrier and a mitochondria activating agent into a myocardial stem cell.
(2) The method according to (1), wherein the complex is a mitochondria-targeting liposome encapsulating the mitochondria activating agent.
(3) The method according to (1) or (2), wherein the complex is a mitochondria-targeting liposome containing
   dioleylphosphatidylethanolamine and
   phosphatidic acid and/or sphingomyelin as constituent lipids of a lipid membrane, and having a mitochondria-targeting molecule on a surface of the lipid membrane, and encapsulating the mitochondria activating agent.
(4) The method according to (3), wherein the mitochondria-targeting molecule is a peptide consisting of an amino acid sequence set forth in SEQ ID NO: 1.
(5) The method according to any one of (1) to (4), wherein the mitochondria activating agent is resveratrol.
(6) A myocardial stem cell produced by introducing a complex of a mitochondria-targeting carrier and a mitochondria activating agent into a myocardial stem cell.
(7) The myocardial stem cell according to (6), wherein the complex is a mitochondria-targeting liposome encapsulating the mitochondria activating agent.
(8) The myocardial stem cell according to (6) or (7), wherein the complex is a mitochondria-targeting liposome containing
   dioleylphosphatidylethanolamine and
   phosphatidic acid and/or sphingomyelin as constituent lipids of a lipid membrane, and having a mitochondria-targeting molecule on a surface of the lipid membrane, and encapsulating the mitochondria activating agent.
(9) The myocardial stem cell according to (8), wherein the mitochondria-targeting molecule is a peptide consisting of an amino acid sequence set forth in SEQ ID NO: 1.
(10) The myocardial stem cell according to any one of (6) to (9), wherein the mitochondria activating agent is resveratrol.
(11) A cell population comprising myocardial stem cells, wherein an average value of ratios of fluorescence intensity of JC-1 dimer to fluorescence intensity of JC-1 monomer when the cell population is stained with fluorescent dye JC-1 is 1 to 4.
(12) A cell preparation for use in treatment and/or prevention of cardiac failure, the cell preparation comprising the myocardial stem cell or cell population according to any one of (6) to (11).
(13) A liposome for use in introduction of an encapsulated substance into mitochondria of a myocardial stem cell, the liposome containing
   dioleylphosphatidylethanolamine and
   phosphatidic acid and/or sphingomyelin as constituent lipids of a lipid membrane, and having a mitochondria-targeting molecule on a surface of the lipid membrane.
(14) The liposome according to (13), wherein the mitochondria-targeting molecule is a peptide consisting of an amino acid sequence set forth in SEQ ID NO: 1.

### Effects of Invention

According to the present invention, there can be provided a myocardial stem cell which is capable of maintaining a therapeutic effect and/or preventive effect by cell transplantation for a prolonged period. The myocardial stem cell can be used for treatment and/or prevention of myocardial injury, recovery, protection or suppression of deterioration of the cardiac function, treatment and/or prevention of cardiac failure, or the like.

### Brief Description of Drawings

Figure 1 is a histogram of flow cytometry showing a myocardial stem cell containing a mitochondria-targeting liposome encapsulating resveratrol and fluorescently labeled with NBD, where the abscissa indicates a fluorescence level of NBD, and the ordinate indicates the number of cells.
Figure 2 shows photographs of a myocardial stem cell observed with a confocal laser scanning microscope (CLSM), the myocardial stem cell containing a mitochondria-targeting liposome encapsulating resveratrol and fluorescently labeled with NBD. Photograph B shows RES-MITO-Porter stained with NBD (green), photograph C shows mitochondria stained with MTDR (red), photograph D shows a cell nucleus stained Hoechst 33342 (blue), photograph A shows photographs B to D superimposed on one another, and the scale bar for each photograph represents a length of 20 µm.
Figure 3 is a graph showing a cell viability under cell injury caused by doxorubicin (final concentration: 10 µg/mL, or 50 µg/mL) in co-culture of a myocardial blast cell and a myocardial stem cell containing a mitochondria-targeting liposome encapsulating resveratrol (MA-Cell) or an untreated myocardial stem cell (CPC), where "co-culture" indicates co-culture of a myocardial blast cell and MA-Cell, "CPC alone" indicates coculture of a myocardial blast cell and CPC, and "control" indicates monoculture of a myocardial blast cell.
Figure 4 shows a cell viability under cell injury caused by doxorubicin (final concentration: 10 µg/mL, 30 µg/mL, or 50 µg/mL) in co-culture of a myocardial blast cell with a myocardial stem cell containing a mitochondria-targeting liposome encapsulating resveratrol (MA-Cell (+RES-MITO-Porter)), a myocardial stem cell containing empty MITO-Porter (CPC (+MITO-Porter)), a myocardial stem cell treated directly with resveratrol (CPC (+RES)), or CPC.
Figure 5 shows a cell viability after elapse of 48 hours under cell injury caused by doxorubicin (final concentration: 10 µg/mL) in co-culture of a myocardial blast cell with a myocardial stem cell containing a mitochondria-targeting liposome encapsulating resveratrol (CPC + RES-MITO-Porter), a myocardial stem cell treated directly with resveratrol (CPC (+RES)), or CPC.
Figure 6 is a graph showing a cell viability under cell injury caused by doxorubicin (final concentration: 10 µg/mL) at each dose of resveratrol in coculture of a myocardial blast cell and MA-Cell.
Figure 7 is a graph showing a Kaplan-Meier curve for MA-Cell- or CPC-transplanted or untreated doxorubicin cardiac failure model mice and healthy mice.
Figure 8 is a graph showing a change in average body weight of MA-Cell- or CPC-transplanted or untreated doxorubicin cardiac failure model mice and healthy mice.
Figure 9 is a graph showing a dihydroethidium (DHE) positive cell ratio in the cardiac tissues of MA-Cell- or CPC-transplanted or untreated doxorubicin cardiac failure model mice and healthy mice.
Figure 10 is a graph showing an apoptosis inductivity in the cardiac tissues of MA-Cell- or CPC-transplanted or untreated doxorubicin cardiac failure model mice and healthy mice.
Figure 11 is a graph showing a left ventricle shortening fraction for MA-Cell-transplanted doxorubicin cardiac failure model mice and healthy mice.
Figure 12 is a graph showing the relative expression levels of the genes: PGC1α, ESRRa, SDHA, Cox1 and ATPla in the cardiac tissues of MA-Cell- or CPC-transplanted or untreated doxorubicin cardiac failure model mice and healthy mice.
Figure 13 is a graph showing a mitochondrial respiratory chain complex formation ratio in the cardiac tissues of MA-Cell- or CPC-transplanted or untreated doxorubicin cardiac failure model mice and healthy mice.
Figure 14 shows photographs showing MA-Cell being engrafted in the mouse heart after transplantation, where the lower left photograph shows myocardial actinin stained with Alexa Flour 488 (green), the upper right photograph shows a cell nucleus stained with Hoechst 33342 (blue), the lower right photograph shows MA-Cell stained with CellVue Claret (red), and the upper left photograph shows these photographs superimposed on one another.

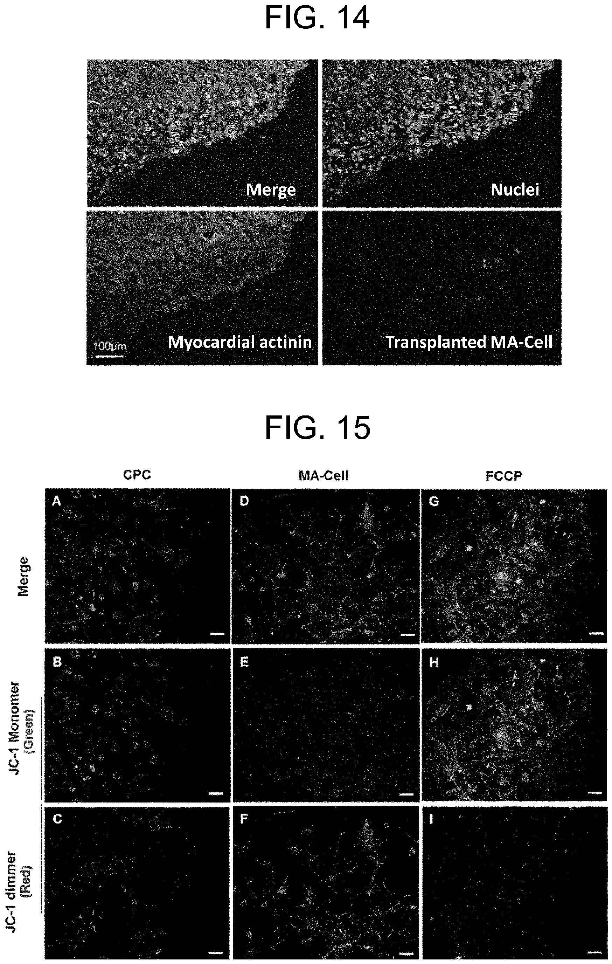

Figure 15 shows photographs showing the results of detecting the mitochondrial membrane potentials of MA-Cell and CPC using fluorescent dye JC-1, where the left photographs show CPC, the central photographs show MA-Cell, the right photographs show CPC to which FCCP has been added, the middle photographs show green fluorescence with a wavelength of 529 nm which corresponds to JC-1 monomer indicating depolarized mitochondria, the lower photographs show red fluorescence with a wavelength of 590 nm which corresponds to JC-1 dimer indicating polarized mitochondria, and the upper photographs show the middle and lower photographs superimposed on one another.
Figure 16 is a graph showing the results of calculating a ratio of fluorescence intensity between JC-1 monomer (green) indicating depolarized mitochondria and JC-1 dimer (red) indicating polarized mitochondria (Dimer/Monomer), on the basis of the image used to detect the mitochondrial membrane potentials of MA-Cell and CPC using fluorescent dye JC-1, where "0" indicates a value for each cell, and "-" indicates an average value (n = 19) .

### Description of Embodiments

A first aspect of the present invention relates to a method for producing a myocardial stem cell for use in treatment and/or prevention of cardiac failure, the method comprising the step of introducing a complex of a mitochondria-targeting carrier and a mitochondria activating agent into a myocardial stem cell.

The myocardial stem cell (also referred to as a cardiac progenitor cell, which is hereinafter referred to as CPC) is a stem cell having a self-replication ability and a differentiation ability to the cardiac muscle, the vascular endothelium, the vascular smooth muscle, the fat, the bone, the cartilage or the like. CPC to be used in the present invention can be separated from a cardiac tissue by a method known to those skilled in the art. One example of the CPC is a cell separated from a cardiac tissue by, for example, Oh et al.'s method (PNAS., 2003, 100, pp. 12313-12318), or Ishigami et al.'s method (Circ Res., 2015, 116, pp. 653-664). CPC differentiated and induced from iPS cells (Funakoshi S. et al., Scientific Reports 6, 2016, 19111) and CPC obtained by reprograming fibroblasts or myocardial cells (Ieda M. et al., Cell, 2010, 142, pp. 375-386) can also be used in the present invention. The documents are hereby incorporated by reference.

The CPC may be one derived from any animal species, but it is preferable to use human CPC when the purpose is to treat or prevent human cardiac failure. In the present invention, it is especially preferable to use CPC separated with the intension of transplantation using self-somatic cells from cardiac tissues of a person suffering from cardiac failure or having a risk of cardiac failure.

The CPC may be one isolated on the basis of expression of a CPC specific marker such as Sca-1, and purified, or may be one contained in a cell population, e.g. a heterogeneous cell population obtained by spheroid culture of cells separated from the heart. In the latter case, the whole cell population is subjected to the step of introducing a complex of a mitochondria-targeting carrier and a mitochondria activating agent as described later, whereby a cell population including CPC having activated mitochondria is produced.

For securing the number of cells necessary for subsequent cell transplantation, the CPC may be used after being grown by performing subculture in vitro as long as the sternness thereof is maintained.

The present invention includes the step of introducing a complex of a mitochondria-targeting carrier and a mitochondria activating agent into CPC.

The mitochondria-targeting carrier is one having a function to selectively reach mitochondria as one of intracellular organelles when the carrier is introduced into a cell. Examples of the mitochondria-targeting carrier may include liposoluble cation substances such as Lipophilic triphenylphosphonium cation (TPP) and Rhodamine 123; polypeptides such as Mitochondrial Targeting Sequence (MTS) peptide (Kong, BW. et al., Biochimica et Biophysica Acta 2003, 1625, pp. 98-108) and S2 peptide (Szeto, H. H. et al., Pharm. Res. 2011, 28, pp. 2669-2679); and mitochondria-targeting liposomes such as DQAsome (Weissig, V. et al., J. Control. Release 2001, 75, pp. 401-408), MITO-Porter (Yamada, Y. et al., Biochim Biophys Acta. 2008, 1778, pp. 423-432), DF-MITO-Porter (Yamada, Y. et al., Mol. Ther. 2011, 19, pp. 1449-1456) and modified DF-MITO-Porter modified with S2 peptide (Kawamura, E. et al., Mitochondrion 2013, 13, pp. 610-614). The documents are hereby incorporated by reference regarding production and use of the carriers in the present invention.

A preferred mitochondria-targeting carrier in the present invention is a mitochondria-targeting liposome, and in particular, MITO-Porter, DF-MITO-Porter or modified DF-MITO-Porter is preferable.

The complex of a mitochondria-targeting carrier and a mitochondria activating agent is a substance having a configuration in which a mitochondria-targeting carrier and a mitochondria activating agent behave in a unified manner regardless of whether chemical bonding, physical encapsulation or the like is used to form the complex. For example, when the liposoluble cation lipid or polypeptide is a mitochondria-targeting carrier, a complex of a mitochondria-targeting carrier and a mitochondria activating agent can be formed by bonding the mitochondria-targeting carrier to the mitochondria activating agent using a chemical method such as covalent bonding or ionic bonding in accordance with, for example, Murphy et al.'s method regarding a liposoluble cation substance (G. F. Kelso et al., J. Biol. Chem., 2001, 276, pp. 4588-4596) or a method regarding Szeto peptide as described in JP2007-503461A.

Further, when the mitochondria-targeting carrier is a liposome, a complex of a mitochondria-targeting carrier and a mitochondria activating agent can be formed by chemically bonding the mitochondria activating agent to a surface of a lipid membrane of the liposome, or physically encapsulating the mitochondria activating agent in the liposome, i.e. an internal space blocked by a lipid membrane.

The complex can be introduced into CPC by a method for introduction of the complex into a cell, which is known for the mitochondria-targeting carrier. The complex may be introduced into a cell by, for example, culturing CPC in an appropriate medium containing the complex, or incubating the complex and CPC in the presence of a known substance capable of accelerating uptake of a substance into a cell, such as lipofectamine or polyethylene glycol.

A preferred example of the step of introducing a complex of a mitochondria-targeting carrier and a mitochondria activating agent into CPC in the first aspect of the present invention is a step of introducing a complex into CPC by incubating CPC and a complex which is a mitochondria-targeting liposome encapsulating a mitochondria activating agent, particularly a complex which is MITO-Porter or DF-MITO-Porter having the surface modified with MTS peptide or S2 peptide and encapsulating a mitochondria activating agent.

The mitochondria activating agent is a substance capable of activating a mitochondrial respiratory chain complex (electron transport system), particularly a substance capable of bringing mitochondria into a polarized state in terms of a membrane potential, and in particular, it is preferable to use a substance capable of bringing mitochondria into a hyperpolarized state. Examples of the mitochondria activating agent may include antioxidants such as resveratrol (3,5,4'-trihydroxytrans-stilbene), coenzyme Q10, vitamin C, vitamin E, N-acetylcysteine, TEMPO, SOD and glutathione, and in particular, resveratrol is preferable.

The resveratrol that is preferably used in the present invention may be one extracted from a plant by a known method, or one chemically synthesized by a known method such as, for example, Andrus et al.'s method (Tetrahedron Lett. 2003, 44, pp. 4819-4822).

The CPC produced by the method according to the first aspect of the present invention is one of additional aspects of the present invention, and can considerably improve the viability of mice receiving doxorubicin as shown in Examples below. Further, the CPC is suitably involved in reduction of oxidative stress, suppression of apoptosis or maintenance of mitochondrial functions in cardiac tissues of mice receiving doxorubicin.

It is clinically known that administration of doxorubicin, a type of anthracycline-based pharmaceutical agent, causes severe myocardial injury, and mice receiving doxorubicin are used as cardiac failure model mice. Therefore, the CPC produced by the method according to the first aspect of the present invention can be used for treatment and/or prevention of myocardial injury, particularly severe myocardial injury, recovery, protection or suppression of deterioration of the cardiac function, treatment and/or prevention of cardiac failure, or the like.

Another aspect of the present invention relates to a cell population including myocardial stem cells, wherein an average value of ratios of fluorescence intensity of JC-1 dimer to fluorescence intensity of JC-1 monomer (fluorescence intensity of JC-1 dimer/fluorescence intensity of JC-1 monomer) when the cell population is stained with fluorescent dye JC-1 is 1 to 4.

Mitochondria generate a proton concentration gradient inside and outside the membrane under the action of respiratory chain complexes existing in the mitochondria, and come into a polarized state in which there is a membrane potential. When receiving apoptosis, metabolic stress or the like, the polarized mitochondria are turned into a depolarized state in which the membrane potential is reduced. In this way, the state of polarization of mitochondria is a parameter indicating a metabolism activity of mitochondria, and a cell having a large number of polarized mitochondria is considered to be a cell having activated mitochondria.

It is known that fluorescent dye JC-1 (5,5',6,6'-tetrachloro-1,1',3,3'-tetraethylbenzimidazolylcarbocyanine iodide), which is a mitochondrial membrane potential probe, is a monomer emitting green fluorescence in depolarized mitochondria, but forms a dimer emitting red fluorescence in polarized mitochondria. Therefore, the ratio of fluorescence intensity between JC-1 monomer and JC-1 dimer is an index indicating a state of polarization of mitochondria. The ratio of fluorescence intensity can be measured by, for example, detecting a fluorescence ratio in accordance with manufacturer's protocol using JC-1 commercially available from Thermo Fisher Scientific, Cosmo Bio Co., Ltd. or the like.

The cell population according to this aspect is a cell population including CPC having activated mitochondria, and the degree of activation of mitochondria of CPC included in the population can be represented by an average value of ratios of fluorescence intensity of JC-1 dimer to fluorescence intensity of JC-1 monomer (fluorescence intensity of JC-1 dimer/fluorescence intensity of JC-1 monomer) when the cell population is stained with JC-1.

The average value of ratios of fluorescence intensity can be determined by measuring a ratio of fluorescence intensity of JC-1 dimer to fluorescence intensity of JC-1 monomer (fluorescence intensity of JC-1 dimer/fluorescence intensity of JC-1 monomer) for each of any number of CPCs, preferably more than 10 and less than 100 CPCs included in the cell population, and calculating an average value of the measured ratios. The average value of ratios of fluorescence intensity of JC-1 dimer to fluorescence intensity of JC-1 monomer in a cell population including CPC having activated mitochondria is more than 1, preferably 1 to 4.

The cell population according to this aspect is a cell population mainly consisting of CPC, preferably a cell population which does not substantially include cells other than CPC. The cell population can be produced typically by the foregoing method according to the first aspect of the present invention.

Both the CPC and the cell population including CPC can be used for treatment and/or prevention of myocardial injury, recovery, protection or suppression of deterioration of the cardiac function, treatment and/or prevention of cardiac failure, or the like. Therefore, still another aspect of the present invention provides a method for treating and/or preventing myocardial injury or cardiac failure, the method comprising the step of administering an effective amount of the CPC or the cell population to a subject in need thereof. Further, still another aspect of the present invention provides a method for recovering the cardiac function, a method for protecting the cardiac function or a method for suppressing deterioration of the cardiac function, the method comprising the step of administering an effective amount of the CPC or the cell population to a subject in need thereof.

Further, still another aspect of the present invention provides a cell preparation having the CPC or the cell population including CPC as an active ingredient, particularly a cell preparation for use in treatment and/or prevention of myocardial injury or cardiac failure, a cell preparation for use in recovery and/or protection of the cardiac function, a cell preparation for use in suppression of deterioration of the cardiac function, or the like.

The cell preparation as one aspect of the present invention can be prepared by a method known to those skilled in the art. For example, the cell preparation can be prepared as a form of a suspension solution obtained by suspending cells in water, other pharmaceutically acceptable buffer solution or the like as necessary. The cell preparation may contain pharmaceutically acceptable additives such as a carrier or medium, e.g. vegetable oil, an emulsifier, a suspending agent, a surfactant, a stabilizer, an excipient and a preservative.

The cell preparation as one aspect of the present invention contains an effective amount of the CPC or the cell population including CPC. The term "effective amount" as used herein means an amount of CPC necessary for exhibiting an effect such as treatment and/or prevention of myocardial injury, recovery, protection or suppression of deterioration of the cardiac function, or treatment and/or prevention of cardiac failure. The effective amount, which depends on the condition of a subject requiring treatment, is, for example, 1 × 10³ cells to 1 × 10⁹ cells, preferably 1 × 10⁶ cells to 1 × 10⁹ cells, more preferably 1 × 10⁷ cells to 1 × 10⁹ cells per individual subject, and the cell preparation may be administered in such an amount once or two or more times at appropriate intervals.

The method for administering the cell preparation is not particularly limited, and examples thereof include administration methods that are commonly used, e.g. intravascular administration (preferably intravenous administration), intraperitoneal administration and local administration. Intravenous administration or local administration to the heart is preferable.

Still another aspect of the present invention provides a liposome for use in introduction of an encapsulated substance into mitochondria of CPC, the liposome containing dioleylphosphatidylethanolamine (DOPE) and phosphatidic acid (PA) and/or sphingomyelin (SM) as constituent lipids of a lipid membrane, and having a mitochondria-targeting molecule on a surface of the lipid membrane. A liposome in which the mitochondria-targeting molecule is a peptide consisting of an amino acid sequence set forth in SEQ ID NO: 1 (i.e., S2 peptide) can be produced by Kawamura et al.'s method described above. The liposome according to this aspect may further have, in addition to S2 peptide, a peptide consisting of an amino acid sequence set forth in SEQ ID NO: 2 (i.e., an octaarginine peptide) on a surface of a lipid membrane, and such a liposome can be produced by a method as described in JP5067733B. The substance to be encapsulated is preferably the mitochondria activating agent described in the first aspect of the present invention.

The present invention will be further described in detail by way of the following Examples, but the present invention is not limited to these Examples.

### Examples

### Example 1. Preparation of CPCs with resveratrol delivered to mitochondria

### (1) Purification of mouse CPC

Mouse CPC was isolated and purified in the following manner in accordance with Oh et al.'s method (PNAS. 2003, 100, pp. 12313-12318).

The heart was excised from an 8-week-old c57BL6/J male mouse, and subjected to collagenase treatment and Percoll density gradient treatment to extract a cell group including CPC. The obtained cell group was subjected to primary culture, sorting was then performed by MACS system to selectively extract Sca-1 positive CPC, and the Sca-1 positive CPC was subjected to subculture to isolate mouse CPC. For the isolated CPC, the amount of a surface marker protein was determined by flow cytometry (FACS), the gene expression levels of a myocardial transcription factor and a structural protein were determined by a PCR method, and the values thereof were confirmed to agree with those reported previously (data not shown).

### (2) Preparation of RES-MITO-Porter modified with S2 peptide

A mitochondria-targeting liposome (RES-MITO-Porter) having the surface modified with S2 peptide and encapsulating resveratrol was prepared in the following manner.

A mixed solution of 137.5 µL of a 1 mM lipid ethanol solution of 1,2-dioleyl-sn-glycero-3-phosphatidylethanolamine (DOPE) and sphingomyelin (SM) (DOPE/SM = 9:2) and 112.5 µL of chloroform was dried under reduced pressure to prepare a lipid membrane film. 250 µL of a 10 mM HEPES buffer solution containing resveratrol in an amount of 2.3 mg per mL was added to the lipid membrane film to hydrate the lipid membrane film (room temperature, 15 minutes), and ultrasonication treatment was then performed with a bath-type sonicator (AU-25C; Aiwa Ika Kogyo K.K.) to prepare a liposome. A Stearyl S2 solution was added to the liposome in an amount of 10% based on the total amount of lipid, and the resulting mixture was incubated at room temperature for 30 minutes to prepare RES-MITO-Porter.

It was confirmed that the prepared RES-MITO-Porter was nanoparticles having an average particle diameter of 121 ± 7 nm, a zeta potential of 49 ± 1 mV and a resveratrol encapsulation ratio of 87 ± 4% and having a positive charge, and maintained particle physical properties even after storage at 4°C for 1 month.

### (3) Introduction of RES-MITO-Porter into CPC

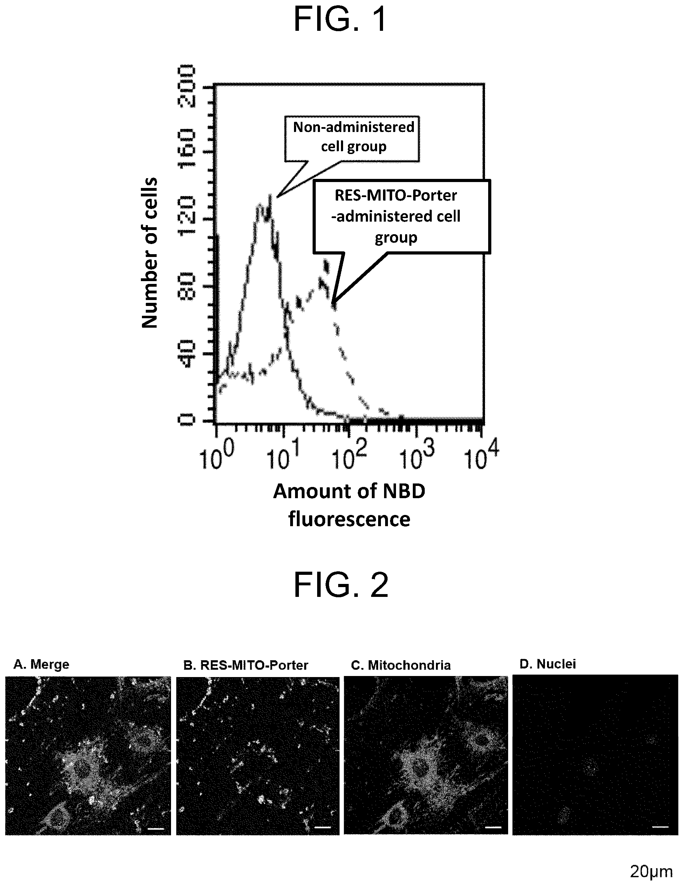

RES-MITO-Porter was labeled with green fluorescent dye NBD (7-nitrobenz-2-oxa-1,3-diazole) in accordance with a previously reported method (Abe, J. et al., J. Pharm. Sci. 2016, 105, pp. 734-740) to evaluate introduction of RES-MITO-Porter into CPC. 200 µL of fluorescently NBD-labeled RES-MITO-Porter with a total amount of lipid of 550 µM (with a resveratrol concentration of 100 µM) was provided, and added to 10 mL of DMEM-F12 medium containing 1 × 10⁶ CPCs, and the resulting mixture was incubated for 1 hour to introduce RES-MITO-Porter into CPC. Using FACS, a fluorescently NBD-labeled carrier taken up in CPC was detected, and thus it was confirmed that RES-MITO-Porter had been introduced into CPC (Figure 1).

Mitochondria of CPC containing fluorescently NBD-labeled RES-MITO-Porter were stained with MTDR in accordance with the previously reported method (Abe, J. et al., J. Pharm. Sci. 2016, 105, pp. 734-740), and then observed with a confocal laser scanning microscope (CLSM), and resultantly, yellow dots were observed at which green NBD and red MTDR overlapped each other. Thus, it was confirmed that RES-MITO-Porter was integrated with mitochondria of CPC (Figure 2).

### Example 2. Myocardial blast cell protection effect of CPC with resveratrol delivered to mitochondria (in vitro experiment)

### (1) Introduction of RES-MITO-Porter into CPC

CPC suspended in DMEM-F12 medium was seeded in a 6-well plate at a density of 1 × 10⁶ cells per well, and cultured at 37°C for 24 hours. RES-MITO-Porter prepared in section (2) in Example 1 was added to each well, and the resulting mixture was incubated for 2 hours to introduce RES-MITO-Porter into CPC for use in subsequent experiments. The CPC containing RES-MITO-Porter is referred to as MA-Cell.

### (2) Cell viability under cell injury caused by doxorubicin

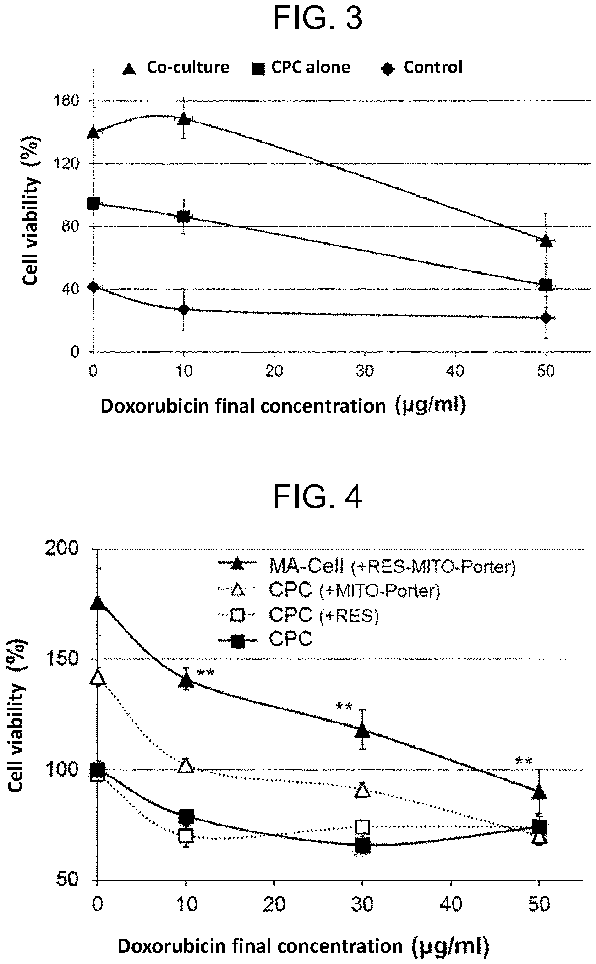

Rat myocardial blast cell, H9c2 cell (purchased from ATCC) and MA-Cell prepared in section (1) were mixed in DMEM-F12 medium in such a manner that the density of H9c2 cell was 3 × 10⁴ cells per well and the density of MA-Cell was 1 × 10⁴ cells per well, and the mixed liquid was subjected to coculture at 37°C for 24 hours. Doxorubicin was added to the coculture liquid in a final concentration of 10 µg/mL (low dose) or 50 µg/mL (high dose) to induce cell injury, and culture was further performed for 16 hours, followed by measuring a cell viability using WST-1 reagent (Takara Bio Inc.). Further, for H9c2 cell to which MA-Cell was not added but only the medium was added (control) and H9c2 cell to which CPC was added instead of MA-Cell (CPC alone), cell injury was induced and a cell viability was measured in the same manner as described above. Figure 3 shows results when the cell viability of non-doxorubicin-treated H9c2 cell is defined as 100%.

MA-Cell was confirmed to suppress a decrease in cell viability under the cell injury action of doxorubicin. Further, when the mixing ratio between H9c2 cell and MA-Cell in coculture was 6:1, the same result was observed (MA-Cell in Figure 4). On the other hand, a decrease in cell viability when instead of MA-Cell, CPC treated directly with resveratrol was added to H9c2 cell (CPC (+RES) in Figure 4) was comparable to a decrease in cell viability when CPC alone was added (CPC in Figure 4), and thus improvement by addition of resveratrol was not observed.

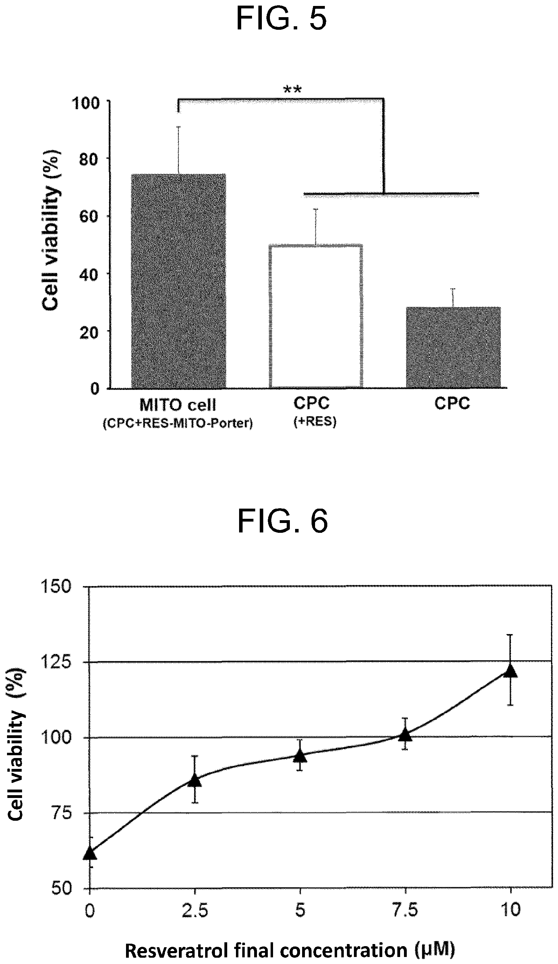

Further, the medium was changed from DMEM-F12 to DMEM High glucose (Thermo), H9c2 cell and MA-Cell, CPC treated directly with resveratrol, or CPC were mixed at a mixing ratio of 6:1, and cocultured at 37°C for 24 hours, doxorubicin was then added in a final concentration of 10 µg/mL to induce cell injury, and culture was further performed for 48 hours, followed by measuring a cell viability. Figure 5 shows results when the cell viability of non-doxorubicin-treated H9c2 cell is defined as 100%. It was confirmed that suppression of a decrease in cell viability under the cell injury action of doxorubicin by MA-Cell was maintained even 48 hours after induction of cell injury.

### (3) Change in cell viability depending on dose of resveratrol

By performing the same operation as described in section (1) above, MA-Cell was prepared while the amount of resveratrol delivered was changed using RES-MITO-Porter having a resveratrol concentration of 0 to 10 µM. The same experiment as described in section (2) above was performed using each MA-Cell and doxorubicin in a final concentration of 10 µg/mL, and a cell viability was measured at each dose of resveratrol. Figure 6 shows results when the cell viability of non-doxorubicin-treated H9c2 cell is defined as 100%.

It was confirmed that a decrease in cell viability under the cell injury action of doxorubicin was improved depending on the dose of resveratrol.

### Example 3. Protective effect against myocardial injury by CPC with resveratrol delivered to mitochondria (in vivo experiment)

### (1) Preparation of doxorubicin myocardial injury model mouse and administration of each CPC

MA-Cell (1 × 10⁶ cells) was transplanted to the heart of each of healthy mice (6 to 8-week-old male C57/BL6 mice) to provide an MA-Cell-transplanted group (n = 6) . 24 hours after the transplantation, myocardial injury was induced by intraperitoneally administering 200 µL of doxorubicin/PBS to the mouse at a level of 25 mg/kg only once with reference to Zhang et al.'s method (Nature Medicine 2012, 18, pp. 1639-1642). In a group of mice which was not subjected to cell transplantation (untreated group) and a group of mice subjected to transplantation of CPC instead of MA-Cell (CPC-transplanted group), myocardial injury was induced in the same manner as described above. Further, for each of the groups, a group of mice which did not receive doxorubicin (healthy group) was provided.

### (2) Viability after induction of myocardial injury

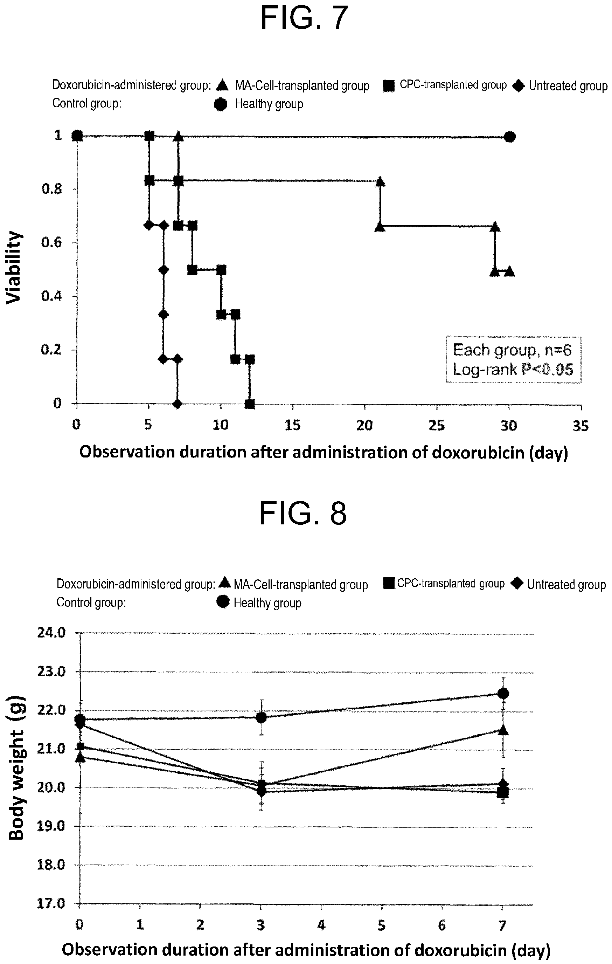

A Kaplan-Meier curve for the viability of each group after induction of myocardial injury was prepared, and statistical processing was performed by log-rank analysis. The results thereof are shown in Figure 7. It was confirmed that the viability of MA-Cell-transplanted group was significantly improved as compared to the viabilities of the untreated group and the CPC-transplanted group.

### (3) Average body weight

Figure 8 shows the mouse average body weights in the groups on the third day and the seventh day after induction of myocardial injury. The body weight temporarily decreased in all the groups of mice receiving doxorubicin, but it was observed that in the MA-Cell-transplanted group the body weight tended to be recovered on the seventh day.

### (4) Oxidative stress condition in cardiac tissue

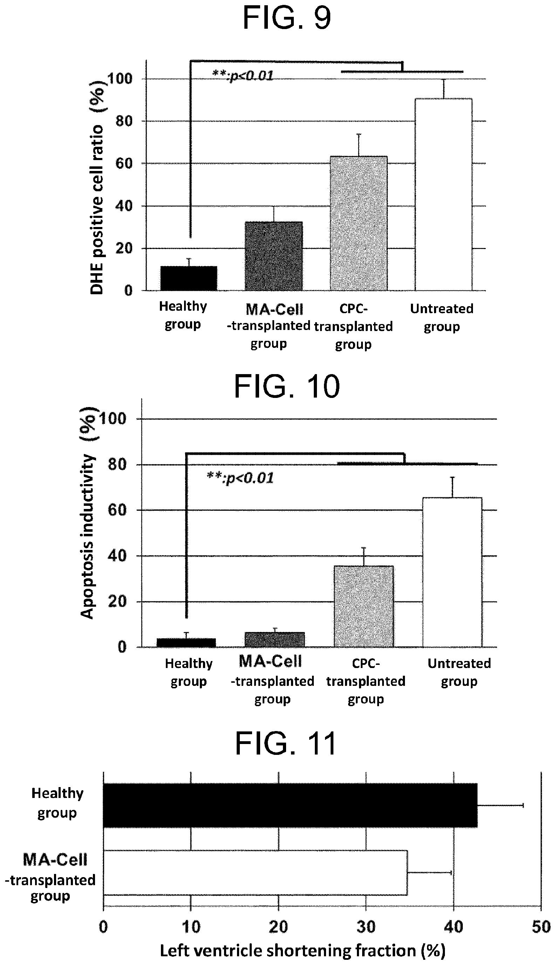

On the third day after induction of myocardial injury, the heart was excised, a cardiac tissue was quickly stained with dihydroethidium (DHE) with reference to the foregoing Zhang et al.'s method, red cells (DHE positive cells) in the cardiac tissue were counted, and the oxidative stress condition of the cardiac tissue was quantitatively determined as a DHE positive cell ratio. The results thereof are shown in Figure 9. The DHE is a fluorescent probe which reacts with active oxygen species in living cells to emit red fluorescence. The untreated group and the CPC-transplanted group exhibited a DHE positive cell ratio significantly higher than the DHE positive cell ratio of the healthy group. On the other hand, it was confirmed that in the MA-Cell-transplanted group, an increase in the number of DHE positive cells tended to be suppressed.

### (5) Apoptosis induction in cardiac tissue

The heart excised in section (4) was subjected to tissue fixation, a TUNEL method was then carried out using TUNNEL In Situ Cell Death Detection Kit, Fluorescein kit (Sigma), and apoptosis positive cells in the cardiac tissue were counted to calculate an apoptosis inductivity. The results thereof are shown in Figure 10. A large number of apoptosis induction cells were observed in the untreated group and the CPC-transplanted group, whereas it was confirmed that in the MA-Cell-transplanted group, apoptosis induction was suppressed to a level comparable to that in the healthy group.

### (6) Cardiac function (left ventricle shortening fraction)

The cardiac function (left ventricle shortening fraction) of each of the mice in the healthy group and the MA-Cell-transplanted group 5 weeks after induction of myocardial injury was measured by carrying out cross-sectional echocardiography. The results thereof are shown in Figure 11. There was no significant difference in left ventricle reduction ratio between the MA-Cell-transplanted group and the healthy group.

### (7) Mitochondrial function

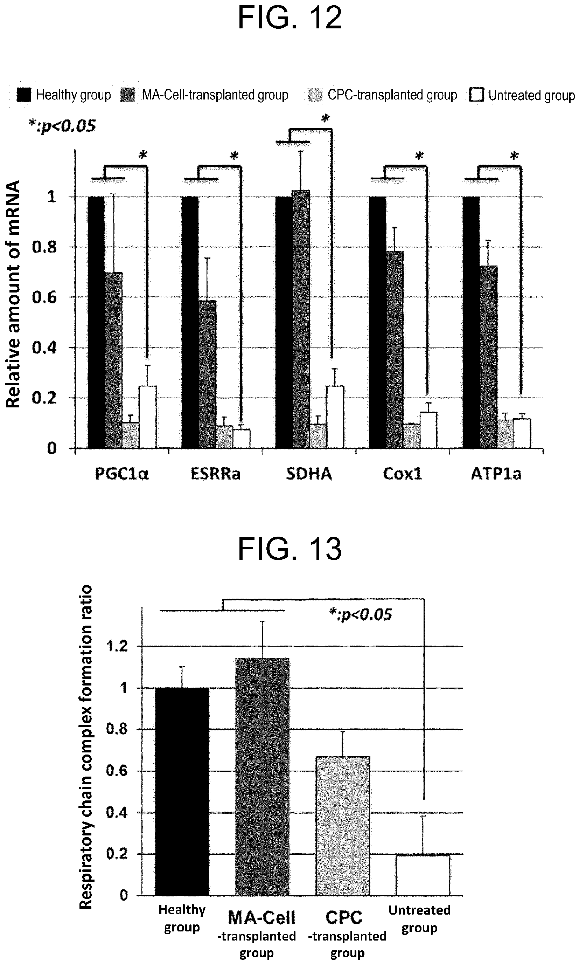

Total RNA was extracted from the cardiac tissue excised in section (4), purified, and subjected to reverse transcription reaction, and a real time PCR method with GAPDH set as an internal standard was then carried out to determine the expression levels of the genes: PGC1α and ESRRa related to mitochondrial neogenesis, and SDHA, Cox1 and ATPla related to the mitochondrial respiratory chain complex. Figure 12 shows relative expression levels in the groups when the expression level of each gene in the healthy group is defined as 1. It was confirmed that the expression level of each gene significantly decreased in the untreated group and the CPC-transplanted group, whereas in the MA-Cell-transplanted group, a decrease in expression level was suppressed. These results showed that in the cardiac tissues of the MA-Cell-transplanted group, expression of mitochondrial neogenesis and mitochondria oxidative phosphorylation gene groups was significantly maintained even after induction of myocardial injury.

### (8) Structure preservation of mitochondrial respiratory chain complex

A protein was extracted from the cardiac tissue excised in section (4), and Blue-Native PAGE was performed. After the electrophoresis, Western-blotting using a mitochondrial Complex I antibody (Abcam) and a mitochondrial Complex II antibody (Abcam) was carried out to quantitatively determine a band in the electron transport system Super-complex band (1,000 kDa). Figure 13 shows Super-complex formation ratios calculated by performing correction with the band quantitative value from the Complex II antibody. It was confirmed that in the CPC-transplanted group and the MA-Cell-transplanted group, the mitochondrial respiratory chain complex structure was preserved as compared to the untreated group. It was confirmed that particularly in the MA-Cell-transplanted group, the higher-order structure of the mitochondrial respiratory complex was preserved at a level comparable to that in the healthy group.

### Example 4. Engraftment of MA-Cell in myocardial tissue

MA-Cell and CPC stained red at the cell membrane surface were prepared using CellVue Claret Far Red (Sigma) in accordance with manufacturer's protocol. Using these cells, cell transplantation and induction of myocardial injury were performed in the same manner as in Example 3, and the heart was excised on the seventh day after induction of injury. The excised heart was stained green at the cardiac muscle using a myocardial actinin antibody (Ms monoclonal anti-sarcomeric α actinin Ab (Sigma)) as a primary antibody and Alexa Fluor 488 Goat F (ab')2 anti-Ms IgG (H+L) (Life Technologies) as a secondary antibody, stained blue at the cell nuclei using Hoechst 33342, and observed with a microscope. Figure 14 shows microscope photographs for MA-Cell-transplanted mice.

The cardiac tissues of the MA-Cell-transplanted mouse were confirmed to have red transplanted cells on green myocardial tissues (positions indicated by the white arrow in Merge on the upper left in Figure 14). On the other hand, red fluorescence was not observed in the CPC-transplanted group (data is not shown).

### Example 5. Mitochondrial membrane potential of MA-Cell

For MA-Cell, and CPC prepared in Example 1, the mitochondrial membrane potential was examined using fluorescent dye JC-1 (Invitrogen) in accordance with manufacturer's protocol. JC-1 emits red fluorescence with a wavelength of 590 nm when accumulating on polarized mitochondria, and JC-1 is diffused into the cytoplasm to emit green fluorescence with a wavelength of 529 nm when mitochondria are depolarized (the membrane potential is eliminated). The results are shown in Figure 15.

In CPC, localized red fluorescence as well as green fluorescence was observed in mitochondria (CPC on the left in Figure 15). Further, when CPC was treated with FCCP, which is an uncoupling agent for the mitochondrial membrane potential, red fluorescence was substantially eliminated, so that only green fluorescence was observed (FCCP on the right in Figure 15). On the other hand, a larger amount of red fluorescence was observed in MA-Cell than in CPC (MA-Cell in Figure 15).

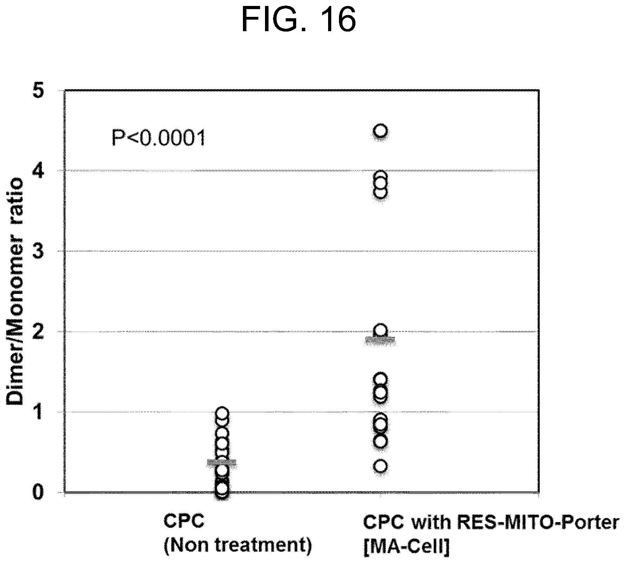

Further, for 19 cells for each of MA-Cell and CPC, a ratio of the fluorescence intensity of polarized mitochondria (red, Dimer) to the fluorescence intensity of depolarized mitochondria (green, Monomer) (Dimer/Monomer ratio) was calculated (Figure 16). The results thereof showed that in MA-Cell, the ratio was in the range of 0.5 to 4.5, with the average value being 1.9, whereas in CPC, the ratio was in the range of 0 to 1, with the average value being 0.4.

## Claims

1. A method for producing a myocardial stem cell for use in treatment and/or prevention of cardiac failure, the method comprising the step of introducing a complex of a mitochondria-targeting carrier and a mitochondria activating agent into a myocardial stem cell.

2. The method according to claim 1, wherein the complex is a mitochondria-targeting liposome encapsulating the mitochondria activating agent.

3. The method according to claim 1 or 2, wherein the complex is a mitochondria-targeting liposome containing
dioleylphosphatidylethanolamine and
phosphatidic acid and/or sphingomyelin as constituent lipids of a lipid membrane, and having a mitochondria-targeting molecule on a surface of the lipid membrane, and encapsulating the mitochondria activating agent.

4. The method according to claim 3, wherein the mitochondria-targeting molecule is a peptide consisting of an amino acid sequence set forth in SEQ ID NO: 1.

5. The method according to any one of claims 1 to 4, wherein the mitochondria activating agent is resveratrol.

6. A myocardial stem cell produced by introducing a complex of a mitochondria-targeting carrier and a mitochondria activating agent into a myocardial stem cell.

7. The myocardial stem cell according to claim 6, wherein the complex is a mitochondria-targeting liposome encapsulating the mitochondria activating agent.

8. The myocardial stem cell according to claim 6 or 7, wherein the complex is a mitochondria-targeting liposome containing
dioleylphosphatidylethanolamine and
phosphatidic acid and/or sphingomyelin as constituent lipids of a lipid membrane, and having a mitochondria-targeting molecule on a surface of the lipid membrane, and encapsulating the mitochondria activating agent.

9. The myocardial stem cell according to claim 8, wherein the mitochondria-targeting molecule is a peptide consisting of an amino acid sequence set forth in SEQ ID NO: 1.

10. The myocardial stem cell according to any one of claims 6 to 9, wherein the mitochondria activating agent is resveratrol.

11. A cell population comprising myocardial stem cells, wherein an average value of ratios of fluorescence intensity of JC-1 dimer to fluorescence intensity of JC-1 monomer when the cell population is stained with fluorescent dye JC-1 is 1 to 4.

12. A cell preparation for use in treatment and/or prevention of cardiac failure, the cell preparation comprising the myocardial stem cell or cell population according to any one of claims 6 to 11.

13. A liposome for use in introduction of an encapsulated substance into mitochondria of a myocardial stem cell, the liposome containing
dioleylphosphatidylethanolamine and
phosphatidic acid and/or sphingomyelin as constituent lipids of a lipid membrane, and having a mitochondria-targeting molecule on a surface of the lipid membrane.

14. The liposome according to claim 13, wherein the mitochondria-targeting molecule is a peptide consisting of an amino acid sequence set forth in SEQ ID NO: 1.
